# EUROPEAN PATENT APPLICATION

(11) **EP 4 008 331 A1**
(43) Date of publication of application: **08.06.2022**
(21) Application number: 21211755.0
(22) Date of filing: 01.12.2021
(51) Int. Cl.: A61K 31/704, A61K 31/4422, A61K 31/575, A61K 31/07, A61K 31/19, A61K 31/728, A61K 36/00, A61P 9/14, A61P 17/02

(54) **PHARMACEUTICAL COMPOSITION FOR USE IN THE TREATMENT OF ANAL FISSURES AND HEMORRHOIDS**

(30) Priority: 02.12.2020 IT 202000029480
(71) Applicant: Teia Research Societa' a Responsabilita' Limitata Semplificata, 80038 Pomigliano d'Arco (NA) (IT)
(72) Inventor: PICCOLO, Federico, 80038 Pomigliano d'Arco NA (IT); GRIECO, Paolo, 80121 Napoli (IT); SPERLONGANO, Rossella, 80038 Pomigliano D'arco NA (IT); FICO, Domenico Ivan, 80078 Pozzuoli NA (IT); DESIDERIO, Vincenzo, 80137 NAPOLI (IT); CARAGLIA, Michele, 83031 ARIANO IRPINO AV (IT)
(74) Representative: Cattaneo, Elisabetta

(57) **Abstract**

The present invention concerns a pharmaceutical composition comprising: nifedipine and escin comprising a content of β-sitosterol ranging from 10% to 17% by weight. The composition of the invention, preferably in the form of cream, ointment, foam, powders or gel for use in the treatment of anal fissures and hemorrhoids, is also described.

## Description

### FIELD OF THE INVENTION

The invention relates to a pharmaceutical composition comprising the active ingredients nifedipine and escin, *i.e.* extract of *Aesculus Hippocastanum*, suitable for use in the treatment of anal fissures and hemorrhoids.

### STATE OF THE ART

Hemorrhoids are defined as cushions of richly vascularized tissue, which line the lower part of the rectum and have the function of completely closing the anal canal, contributing to fecal continence.

Therefore, the term hemorrhoids refers to an anatomical part of the human body and not, as mistakenly often it is heard, a pathology. The physiological role of hemorrhoids is to participate in the mechanism of fecal evacuation and continence. However, when for various reasons there is a dilatation and loss of elasticity of the vascular walls of the hemorrhoidal plexus, such as to determine the characteristic symptoms of this pathology, then we can speak of hemorrhoidal disease. Hemorrhoidal disease is the most widespread proctological disease in the world, given that it is estimated that 3 out of 5 people are affected in the Western countries, although it is difficult to establish the real incidence as only a minority of patients consult a doctor and often non-specific anal symptoms are attributed to hemorrhoidal disease.

In Italy, the incidence is of about 1,000,000 new cases per year (2% of the population) and about 35,000 surgical interventions are performed each year for hemorrhoidal disease.

Hemorrhoids occur more often between the ages of 45 and 65 and, as far as their appearance is concerned, apparently there aren't any differences between the sexes.

Alongside hemorrhoids, it is possible to associate another pathology defined with the term anal fissure, clinically defined as a wound, usually a linear or oval ulceration, a few millimeters long and located at the level of the external mucocutaneous margin of the anus.

It can occur at any age and affects male and female sex equally. In 85-90% of cases it is a single and solitary lesion usually located in the posterior portion, while in 10-15% of cases it is located at the level of the anterior midline.

Fissures located in other regions of the anal mucocutaneous margin should raise the suspicion for other diseases that require further investigation.

Fissures cause very intense pain and bleeding on defecation, they are quite common in the population and for this reason they are often mistaken for diseases having similar symptoms such as hemorrhoids. There is no specific age of onset since they are relatively common in newborns, although the greatest incidence occurs in youth and adults.

For the treatment of hemorrhoids, various topical preparations (creams, pastes, ointments and suppositories) are currently available on the market intended for the symptomatic treatment of hemorrhoids. The active ingredients contained therein include astringent substances (for example, basic bismuth gallate and zinc oxide), local anesthetics (for example, tetracaine and lidocaine), corticosteroids (for example, hydrocortisone and fluocortolone), alone or in association. These products provide immediate relief, reducing pain and inflammation, without being a permanent solution to the underlying disease. For all these products, the continuous use for periods of time longer than 5-7 days is contraindicated as they can cause sensitization of the anal skin.

On the other hand, regarding the pharmacological treatment of fissures, about 30% of the cases can be resolved with a local treatment based on anesthetic creams applied several times a day, especially before defecation, to attenuate the spasm of the anal sphincter.

In addition, in cases of constipation, mild laxatives, or drugs that increase and soften the fecal mass, can be useful to make defecation easier.

Some anesthetic creams containing nitroglycerin, a drug also used in angina pectoris attacks, can give good results, but the onset of headache that often follows tends to limit their use.

Some clinical data also suggested the use of direct injection of botulinum toxin to relax the anal sphincter, but the costs and expectations of clinical success have reduced the use of this practice.

The need is therefore still felt to provide effective treatments for the treatment of hemorrhoids and anal fissures.

### SUMMARY OF THE INVENTION

The above object was achieved by a pharmaceutical composition comprising:
- Nifedipine; and
- Escin comprising a content of β-sitosterol ranging from 10% to 17% by weight. The inventors of the present invention have in fact surprisingly discovered that the association of a known active ingredient with a substance of natural origin, namely the extract of *Aesculus Hippocastanum* called escin, made it possible to treat hemorrhoids and anal fissures effectively.

In an advantageous embodiment, the composition of the invention comprises an amount of nifedipine of about 0.3% by weight, and an amount of escin of about 2% by weight, with respect to the weight of the final composition.

The main innovation of the product lies in the combination of appropriate and defined active ingredients that act synergistically not only for symptomatic but also for resolutive treatment of hemorrhoids and anal fissures.

Under another aspect, therefore, the invention comprises the composition of the invention for the treatment of hemorrhoids and anal fissures.

### DETAILED DESCRIPTION OF THE INVENTION

The invention therefore concerns a pharmaceutical composition comprising:
- Nifedipine; and
- Escin comprising a content of β-sitosterol ranging from 10% to 17% by weight. In an advantageous embodiment, the composition of the invention comprises an amount of nifedipine ranging from 0.15% to 0.5%, preferably about 0.3% by weight, and an amount of escin from 1 to 3%, preferably about 2% by weight, with respect to the weight of the final composition.

One of the active ingredients of the composition is nifedipine having the formula 3,5-dimethyl 2,6-dimethyl-4-(2-nitrophenyl)-1,4-dihydropyridine-3,5-dicarboxylate. Nifedipine is a substance having antihypertensive properties and belongs to the pharmacological class of calcium blockers and to the chemical class of 1,4-dihydropyridines.

Nifedipine is therefore a calcium-antagonist dihydropyridine which, by binding to the calcium ion channels present on cell membranes, prevents the entry of this ion into the cell.

Without being bound to any theory, the inventors believe that the composition of the invention comprising nifedipine, which regulates the increase in calcium concentration inside the cell and therefore is essential for cell contraction, blocking its intracellular entry and therefore generating a smooth muscle relaxation with consequent physiological relief, reduces hypertonicity and contraction of the anal sphincter smooth muscle, thus allowing the treatment of hemorrhoids and anal fissures.

The composition of the invention includes Escin, an extract of *Aesculus Hippocastanum*, comprising an amount of β-sitosterol ranging from 10% to 17% by weight, as determined by TLC. Preferably, escin in the form of an extract comprises from 12.5% to 15.5% by weight of β-sitosterol, as determined by TLC. In an advantageous embodiment, the composition of the invention comprises escin in the form of phytosome or powders comprising an amount of escin of at least 95%, as measured by potentiometry.

Escin is a mixture of saponins, substances having an anti-inflammatory, vasoprotective, and vasoconstrictive activity.

Escin is a natural extract deriving mainly from bark (1%) and leaves (0.2%) but above all from the seeds (13%) of horse chestnut (*Aesculus Hippocastanum*)*.*

Escin is none other than a mixture of raw saponins, among which the most important is escigenin, chemically definable as a pentacyclic triterpene, which is obtained by hydrolysis of the mixture. Taken as a whole, escin is responsible for vasoprotective effects.

The effect of escin on circulatory system disorders is due both to its transformation into the active anti-inflammatory component and to a direct stimulation of the excretion of glucocorticoids which inhibit the inflammatory process and the formation of edema caused by hemorrhoids and fissures.

Escin, in fact, simultaneously performs an anti-inflammatory action on the circulatory system, a vasoprotective and vasoconstrictive actions on blood vessels and, finally, a blood thinning action.

Without being bound to any theory, the inventors believe that, by comprising escin, the composition is able to intervene on the local electrolyte exchange, counteracting the formation of edema: increasing the resistance of the capillaries, reducing the vascular permeability, and thus eliminating accumulated interstitial liquids.

The composition, which comprises escin having anti-edema properties, has therefore a beneficial effect on circulation, justifying its use in the treatment of swelling, heavy feeling, fatigue, or lymphatic stagnation, specifically in the presence of varicose veins and postphlebitic syndromes, in the case of hemorrhoids.

In an advantageous embodiment, the composition of the invention comprises an amount of nifedipine ranging from 0.15% to 0.5% by weight, and an amount of escin from 1 to 3% by weight, with respect to the weight of the final composition. In an advantageous embodiment, the composition of the invention comprises an amount of nifedipine of about 0.3% by weight, and an amount of escin of about 2%. Preferably the composition of the invention comprises nifedipine and escin in a weight ratio of 0.2-0.4 (nifedipine) : 1.0-3.0 (escin), preferably of 0.3: 2.0.

In addition to the active ingredients, nifedipine and escin, additional substances may be present to improve compliance.

The composition of the invention may therefore include one or more substances selected from the group consisting of Zanthalene, Hyaluronic Acid, Glycyrrhetic Acid or Enoxolone.

Zanthalene is a natural active ingredient (Ecocert approved), consisting of a lipophilic solution containing about 20% of the extract obtained from fruit peels of *Zanthoxylum Bungeanum*, a perennial plant very widespread in Asia and widely used in Chinese traditional medicine and gastronomical tradition. The extract contains hydroxy alpha- and beta-sanshools alkylamides. This natural active ingredient can advantageously be present in the composition of the invention, resulting in the latter having also a soothing, calming, and anti-itching action in the treatment of anal fissures and hemorrhoids.

Zanthalene is preferably present in the composition in an amount in the range from 0.3% to 0.8% by weight, with respect to the weight of the final composition.

The composition of the invention may also comprise Vitamin A.

Vitamin A is known to reduce the onset of infections in wounds, as it improves the growth activity of leukocytes, thus intervening to increase the white blood cells that are activated in destroying bacteria. The composition of the invention also includes vitamin A which stimulates the synthesis of collagen in the wound, allowing faster healing of fissures and hemorrhoids.

Vitamin A is preferably present in the composition in an amount in the range from 0.01 to 0.05% by weight, with respect to the weight of the final composition. Advantageously, the composition of the invention may also include hyaluronic acid, which, being a natural component of the skin, enables the composition to stimulate healing recovery phenomena.

Hyaluronic acid is preferably present in the composition in an amount in the range from 1 to 5% by weight, with respect to the weight of the final composition.

Glycyrrhetic acid (18 β-glycyrrhetic acid) is a pentacyclic triterpene acid that is obtained from the licorice plant (Glycyrrhiza glabra), a spontaneous perennial plant grown in many places, especially in the southern provinces of Italy, in Spain and Asia Minor. In addition to exerting a flavoring action, this component is a powerful skin maintenance agent. Studies have shown that glycyrrhetic acid has anti-inflammatory, hypoallergenic, antiviral, and anti-ulcer properties. The anti-inflammatory effects appear to be linked to the chemical similarity with cortisone and have already been applied in numerous clinical contexts, for example in the treatment of chronic hepatitis. Glycyrrhetic acid has also been shown to have good antibacterial activity against various bacterial strains, including *Staphylococcus aureus*, probably thanks to the inhibition of some metabolic pathways of carbohydrates and amino acids. The composition of the invention which comprises glycyrrhetic acid also has an anti-inflammatory activity in the treatment of hemorrhoids and anal fissures.

Glycyrrhetic acid is preferably present in the composition in an amount in the range from 0.8 to 2.0% by weight, with respect to the weight of the final composition. The composition may comprise additives useful for its final formulation.

The composition to be administered may be prepared with any pharmaceutically acceptable component/excipient. Hydroxyethylcellulose can be mentioned among excipients.

The composition according to the invention is therefore preferably formulated to generate a product for topical use only.

More preferably, the pharmaceutical formulation comprising the composition of the invention is in the form of a gel, even more preferably for use by topical anal administration.

The invention therefore concerns the composition of the invention for use as a medicament.

The medicament formulation can be administered via any route of administration, preferably via topical administration, more preferably in the form of cream, ointment, gel, powders, or foam.

More preferably, the composition of the invention is administered as a preparation in the form of a unit dose, preferably topical, for example as an ointment or gel, comprising as active ingredients 0.3 mg of Nifedipine, 2 mg of escin per 100 mg of finished product. Said composition in unit dose form advantageously and preferably also comprises 0.5 mg of Zanthalene, 0.01 mg of Vitamin A, 3.0 mg of Hyaluronic acid, 1.0 mg of glycyrrhetic acid or enoxolone per 100 mg of finished product.

Under another aspect, the invention concerns the composition comprising said composition for use in the treatment of anal fissures and hemorrhoids.

All the preferred and advantageous forms indicated above for the composition are the same for the use of the composition in the treatment of anal fissures and hemorrhoids.

The following are some examples of implementation of the invention and evaluation of the synergy deriving from the composition of the invention, provided by way of non-limiting example of the same invention.

### EXPERIMENTAL PART

### Example 1: Preparation of the composition of the invention

For preparing the composition of the invention and then the final product, such as a medicament for topical use, the following was used
- 0.3 mg of Nifedipine
- 2.0 mg of escin phytosome sold by Indena under the name Escin β-sitosterol Phytosome^{®}
- 0.5 mg of Zanthalene
- 3.0 mg of Hyaluronic Acid
- 1.0 mg of 18 β-Glycyrrhetic Acid
- 0.01 mg of Vitamin A

Hydroxymethylcellulose was added as excipient and water to reach 100 g.

The above ingredients were used to formulate a product to be administered by topical route in the form of a gel.

### Example 2: Evaluation of the activity of the preparation of the invention

The Formulation, as prepared in Example 1, was tested in the treatment of anal fissures and hemorrhoids as compared to the two active ingredients nifedipine and escin used individually.

For evaluating the effect of the Formulation, as prepared in Example 1, an animal model was used as described by Yildirim et al (Apaydin Yildirim B, Aydin T, Kordali S, Yildirim S, Cakir A, Yildirim F. Antihemorrhoidal activity of organic acids of Capsella bursa-pastoris on croton oil-induced hemorrhoid in rats. J Food Biochem. 2020 Jun 25:e13343. doi: 10.1111/jfbc.13343. Epub ahead of print. PMID: 32588462.). In this model, the hemorrhoidal disease is induced by local administration of croton oil.

40 female Sprague-Dawley rats, weighing between 200 and 250 g, were divided into four groups:
Group 1: Croton oil
Group 2: Croton oil +0.3 mg of nifedipine
Group 3: Croton oil +2.0 mg of escin
Group 4: Croton oil + Formulation as prepared in Example 1

Hemorrhoids were induced to all groups by applying the preparation of Croton oil (deionized water, pyridine, diethyl ether and 6% croton oil in diethyl ether in the ratio of 1: 4 : 5 : 10). The animals were then fasted overnight, and subsequently sterile cotton swabs (4 mm diameter) soaked in 100 µL of croton oil preparation were inserted into the anus (rectoanal portion, 20 mm from the anal opening) of all study animals and kept for 10 seconds. A linear development of edema was observed up to 7-8 hours after the application of croton oil. Quantitative evaluation of croton oil-induced plasma exudation in the rats' rectal tissue was determined by estimating the amount of Evans Blue (EB) dye. The EB dye (30 mg/kg) was injected through the tail veins of the animals, 30 minutes before the application of the croton oil preparation to induce hemorrhoids. Twenty-four hours after induction, the animals of the respective groups were treated for 5 days with the formulations indicated above. On the fifth day, all animals were sacrificed and their rectal tissues (20 mm long) isolated and weighed, and the EB dye present in the tissue was extracted using 1 mL of formamide. Sample absorbance was recorded at 620 nm and quantified using the EB dye standard curve.

Groups 2,3 and 4 values thus obtained were analyzed and compared to Group 1 through one-way ANOVA, followed by Dunnett's post-test. Group 2 showed an 8% reduction with respect to Group 1; Group 3 showed a 22% reduction with respect to Group 1; Group 4 showed a 62% reduction with respect to Group 1.

The composition of the invention resulted therefore to be more effective than the comparative compositions.

## Claims

1. A pharmaceutical composition comprising:
- Nifedipine, and
- escin comprising a content of β-sitosterol in the range from 10% to 17% by weight.

2. The composition according to claim 1, wherein the composition of the invention comprises an amount of nifedipine ranging from 0.15% to 0.5%, and an amount of escin from 1 to 3%, with respect to the weight of the final composition.

3. The composition according to claim 2, wherein the composition of the invention comprises an amount of nifedipine of about 0.3% by weight and an amount of escin of about 2% by weight, with respect to the weight of the final composition.

4. The composition according to any one of claims 1 to 3, wherein nifedipine and escin are in a ratio by weight of 0.2 - 0.4(nifedipine) : 1.0-3.0(escin).

5. The composition according to claim 4, wherein nifedipine and escin are in a ratio by weight of 0.3:2.0.

6. The composition according to any one of claims 1 to 5, wherein escin comprises a content of β-sitosterol ranging from 12.5% to 15.5% by weight.

7. The composition according to any one of claims 1 to 6, wherein said composition comprises one or more substances selected from the group consisting of Zanthalene, Vitamin A, Hyaluronic Acid and 18 β-Glycyrrhetic Acid.

8. The composition according to claim 7, wherein Zanthalene is present in the composition in an amount in the range from 0.3% to 0.8% by weight, with respect to the weight of the final composition.

9. The composition according to claim 7, wherein vitamin A is preferably present in the composition in an amount in the range from 0.01% to 0.05% by weight, with respect to the weight of the final composition.

10. The composition according to claim 7, wherein hyaluronic acid is preferably present in the composition in an amount in the range from 1% to 5% by weight, with respect to the weight of the final composition.

11. The composition according to claim 7, wherein glycyrrhetic acid is present in the composition in an amount in the range from 0.8% to 2.0% by weight, with respect to the weight of the final composition.

12. A composition according to any one of claims 1 to 9 for use as a medicament.

13. The composition for use according to claim 12 for use by topical administration, preferably in the form of cream, ointment, gel, powders, or foam.

14. The composition for the use of claim 12 or 13, wherein the composition is administered as a preparation in the form of a unit dose comprising
- 0.3 mg of Nifedipine
- 2.0 mg of escin phytosome
- 0.5 mg of Zanthalene
- 3.0 mg of Hyaluronic Acid
- 1.0 mg of 18 β-Glycyrrhetic Acid, and
- 0.01 mg of Vitamin A

15. A composition comprising nifedipine and escin for use in the treatment of anal fissures and hemorrhoids.
